# EUROPÄISCHE PATENTSCHRIFT

(11) **EP 0 941 076 B1**
(45) Veröffentlichungstag und Bekanntmachung des Hinweises auf die Patenterteilung: **20.04.2005**
(21) Anmeldenummer: 97910432.0
(22) Anmeldetag: 10.10.1997
(51) Int. Cl.: A61K 9/70

(54) **SIEGELMEDIUM FÜR VERBUNDPACKSTOFFE**
SEALING MEDIUM FOR COMPOSITE PACKAGING MATERIALS
SUBSTANCE DE SCELLAGE POUR MATERIAUX D'EMBALLAGE COMPOSITES

(30) Priorität: 29.11.1996 DE 19649534
(43) Veröffentlichungstag der Anmeldung: 15.09.1999
(73) Patentinhaber: LTS Lohmann Therapie-Systeme AG, 56626 Andernach (DE)
(72) Erfinder: KOCH, Reinhard, D-53489 Sinzig (DE); MÜLLER, Frank, A-6833 Klaus/Vorarlberg (AT); FRIIES, Jorgen, DK-5269 Odense S (DK)
(74) Vertreter: Flaccus, Rolf-Dieter, Dr.
(86) Internationale Anmeldenummer: PCT/EP1997/005588
(87) Internationale Veröffentlichungsnummer: WO 1998/023265

(56) Entgegenhaltungen:
- EP-A- 0 563 507
- WO-A-94/04109
- US-A- 4 699 792
- US-A- 5 449 552

## Beschreibung

Die Erfindung betrifft ein Siegelmedium für Verbundpackstoffe, insbesondere zum Verpacken von transdermalen therapeutischen Systemen (TTS) mit teilweise flüchtigen Wirkstoffen.

Siegelmedien zur Herstellung von Verbundpackstoffen der genannten Art sind bekannt. Sie müssen bezüglich ihrer Eigenschaften so ausgewählt sein, daß sie beispielsweise keine signifikante Wirkstoffaufnahme aus dem TTS aufweisen. Mögliche Wechselwirkungen mit aktiven Stoffen oder Hilfsstoffen aus dem TTS sind streng zu vermeiden oder möglichst weitgehend zu reduzieren. Weiterhin sollen die Schichtdicken solcher Medien möglichst gering sein, weil bei hohen Schichtdicken in vielen Fällen eine erhöhte, nicht erwünschte Wechselwirkung zwischen Produkt und Verpackung infolge Migration und Penetration beobachtet wird.
Vergleichsweise dicke Schichten eines Siegelmediums sind auch deshalb von Nachteil, weil für deren Aktivierung beim kurzfristigen haftklebenden Aufschmelzprozeß eine vergleichsweise hohe Zufuhr und Einwirkungszeit von Wärme für die Aktivierung der Siegelschicht benötigt wird.
Zur Erfüllung dieser Forderungen wurden bisher hochwertige Verbundpackstoffe verwendet und die dort eingesetzten Siegelmedien mußten aufgrund der existierenden Herstellungsverfahren in relativ hohen Schichtdicken zwischen 20 und 60 Mikron bei mindestens 20 g/m² aufgetragen werden. Durch derartig hohe Schichtdicken kommt es zu den genannten Nachteilen.

US-A-5 449 552 offenbart eine mehrlagige Verbund-Folie, die eine Heißsiegelschicht in Form einer Folie enthält, welche aus einem Polyolefin oder einem amorphen Polyester besteht und vorzugsweise eine Dicke von 15 bis 150 µm aufweist. Ein in flüssiger Phase vorliegendes Siegelmedium wird in diesem Dokument nicht beschrieben.

Der Erfindung liegt die Aufgabe zugrunde, ein Siegelmedium bereitzustellen, durch dessen Einsatz die vorerwähnten Nachteile und Schwierigkeiten bei der Herstellung von Packmitteln für wirkstoffhaltige Pflastersysteme vermieden werden, welches bei Auftrag in extrem dünner Schicht eine ausreichend hohe Klebkraft entwickelt, in der Konsistenz einer mit üblichen Druckmaschinen verarbeitbaren Druckfarbe vorliegt, aufgrund seiner chemischen Zusammensetzung keine signifikante Wirkstoffaufnahme zuläßt, insbesondere gegen flüchtige Wirkstoffe wie Nikotin eine Barrierefunktion entwickelt und mit einfachen Verfahren, beispielsweise ohne aufwendige Trocknung eines Kaschierklebstoffe oder Aufschmelzung einer vergleichsweise dicken Siegelfolie problemlos anwendbar ist.

Die Lösung der Aufgabe gelingt bei einem Siegelmedium der im Oberbegriff des Anspruchs 1 genannten Art gemäß der vorliegenden Erfindung dadurch, daß das Siegelmedium ein Heißsiegellack ist, der in flüssiger Phase vorliegt und eine Ethylen-Methacrylsäure-Copolymer-Dispersion enthält oder eine Ethylen-Methacrylsäure-Copolymer-Dispersion ist, und bei dem sich keine messbare Wirkstoffaufnahme ergibt. Dieser in flüssiger Phase vorliegende Heißsiegellack ist zum Auftragen von extrem dünnen Siegelschichten in Druckverfahren, zum Beispiel auf partielle Bereiche von Verbundpackstoffen, geeignet.

Die Aufgabe wird ferner durch die Verwendung eines Siegelmediums zur Herstellung von Verbundpacksystemen, insbesondere zum Verpacken von transdermalen Therapiesystemen (TTS) gelöst, wobei nach der Erfindung ein flüssiger Heißsiegellack bereitgestellt wird, der eine Ethylen-Methacrylsäure-Copolymer-Dispersion enthält oder eine Ethylen-Methacrylsäure-Copolymer-Dispersion ist, und bei dem sich keine messbare Wirkstoffaufnahme ergibt. Dieser flüssige Heißsiegellack wird zur Erzeugung einer Siegelschicht in einem Druckverfahren auf einen Verbundpackstoff aufgetragen, wobei das Flächengewicht 1 bis 15 g/m² beträgt. Anschließend wird das Siegelmedium durch einen Aufschmelzprozeß aktiviert und eine haftklebende Schicht ausgebildet.

Durch die Erfindung wird erreicht, daß der Siegellack infolge seiner geringen Schichtdicke keine signifikante Wirkstoffaufnahme zuläßt. Auch ist durch die Möglichkeit, den Siegellack nach der Erfindung im Druckverfahren partiell auf Bereiche von Packstoffen aufzutragen, eine weitere Reduzierung des Mengeneinsatzes und damit der Materialkosten ebenso wie mögliche Wechselwirkungen mit Wirkstoff eines eingepackten Pflasters gegeben.
Der geringe Einsatz von Siegelmedium ergibt sowohl in ökologischer als auch in ökonomischer Hinsicht Vorteile nicht nur bei der Herstellung einer Pflasterverpackung, sondern auch bei deren Entsorgung. Weiterhin erleichtert die Auftragung in Druckverfahren eine exakte partielle Verwendung des Siegellackes nur im Siegelbereich und verringert dadurch Wechselwirkungen zwischen Produkt und Packstoff.
Andererseits sind durch partielle Verwendung des Siegellackes nur im Siegelbereich Verpackungssysteme denkbar, in denen gewünschte Wechselwirkungen, beispielsweise bei Feuchtigkeitsabsorbern, zwischen Produkt und Verpackung wunschgemäß gestaltet werden können. Dagegen bildeten bei früher eingesetzten, vollflächigen Siegelschichten die Filme oder Folien stets die erste, das Produkt vollständig umschließende Schicht einer Verpackung.

Weitere Ausgestaltungen der Erfindung sind entsprechend den Unteransprüchen vorgesehen. Dabei ergibt sich eine Optimierung aus ökologischer und ökonomischer Sicht infolge der geringen aufzubringenden Menge von Siegellack mit Hilfe üblicher einfacher Druckmaschinen sowie aus der Minimierung der für diese Einsatzzwecke meistens sehr teuren Rohstoffe, dies sowohl bei der Herstellung der Packstoffe als auch bei ihrer Entsorgung.

Die Erfindung ermöglicht es, daß damit auf Packstoffbereichen aufbringbare Siegelschichten Flächengewichte zwischen 1 und 15 g/m², bevorzugt Flächengewichte zwischen 2,5 und 3,5 g/m² aufweisen.

Weiter sieht die Erfindung vor, daß das Siegelmedium eine Ethylen-Methacrylsäure-Copolymer-Dispersion ist oder enthält, und daß es aufgrund seiner chemischen Zusammensetzung keine meßbare Wirkstoffaufnahme ergibt.
Aufgrund seiner chemischen Zusammensetzung besitzt es gegenüber flüchtigen Wirkstoffen, insbesondere Nikotin, eine vorteilhafte Barrierewirkung.
Weiterhin ist es mit großem Vorteil beim Vorliegen in Form einer extrem dünnen Siegelschicht bei der Ausbildung einer haftklebenden Schmelzphase unter vergleichsweise äußerst geringer Zufuhr und Einwirkungszeit von Wärme aktivierbar. Einerseits wird dadurch Energie gespart, andererseits kann die Produktionsgeschwindigkeit vorhandener Produktionssysteme zur Herstellung unterschiedlicher Packstoffe und Packmittel wesentlich erhöht und damit die Produktivität signifikant verbessert werden.

Schließlich wird mit der Erfindung erreicht, daß das Siegelmedium nach Aktivierung und Ausbildung einer haftklebenden Schicht Haftkräfte aufweist, die im Festigkeitsbereich der damit verbindbaren Packstoffe liegen.

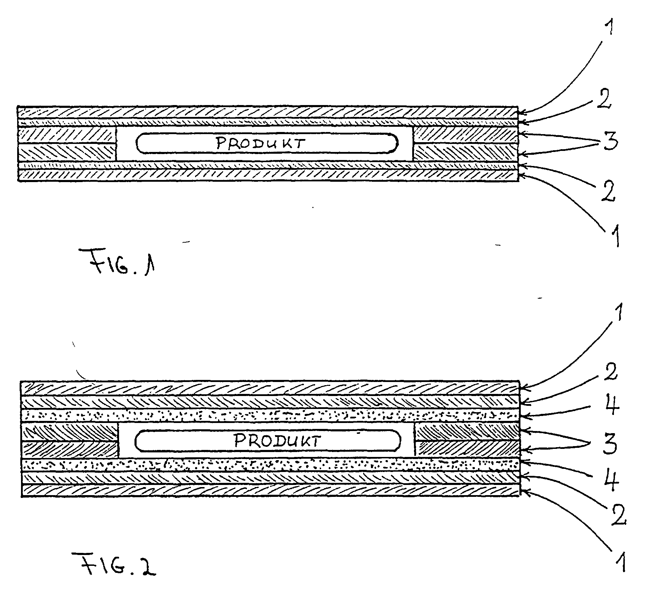

In den Figuren 1 und 2 sind mit einem Siegelmedium nach der Erfindung hergestellte Packungen für wirkstoffhaltige TTS gezeigt.
Figur 1 zeigt eine Packung mit je einer oberen und unteren Trägerschicht 1 sowie einer oberen und unteren Barriereschicht, beispielsweise einer Aluminiumfolie, sowie auf die Barriereschichten 2 partiell aufgebrachten Siegellackschichten 3.
Figur 2 zeigt eine etwas andere Ausgestaltung der Packung mit je einer oberen und unteren Trägerschicht 1, Barriereschichten 2, beispielsweise eine Aluminiumfolie, darunter je ein flächiges, mit dem Produkt in Wechselwirkung befindliches Verpackungselement 4, beispielsweise ein Feuchtigkeitsabsorber, und schließlich partiell aufgebrachte Siegellackschichten 3. Die Erfindung ermöglicht in sowohl besonders ökonomischer als auch besonders ökologischer Weise sowohl die Herstellung als auch die Entsorgung spezieller Verpackungen für TTS, insbesondere solchen mit flüchtigen Wirkstoffen und erfüllt in optimaler Weise die eingangs gestellte Aufgabe.

## Patentansprüche

1. Siegelmedium für Verbundpacksysteme, insbesondere zum Verpacken von transdermalen therapeutischen Systemen (TTS) mit flüchtigen Wirkstoffen, **dadurch gekennzeichnet, daß** das Siegelmedium ein Heißsiegellack ist, der in flüssiger Phase vorliegt und eine Ethylen-Methacrylsäure-Copolymer-Dispersion enthält oder eine Ethylen-Methacrylsäure-Copolymer-Dispersion ist, bei dem sich keine messbare Wirkstoffaufnahme ergibt.

2. Verwendung eines Siegelmediums zur Herstellung von Verbundpacksystemen, insbesondere zum Verpacken von transdermalen Therapiesystemen (TTS), **gekennzeichnet durch**
- das Bereitstellen eines flüssigen Heißsiegellacks, der aus einer Ethylen-Methacrylsäure-Copolymer-Dispersion besteht oder eine Ethylen-Methacrylsäure-Copolymer-Dispersion enthält, bei der sich keine messbare Wirkstoffaufnahme ergibt,
- das Auftragen des flüssigen Heißsiegellacks in einem Druckverfahren zu einer Siegelschicht auf einem Verbundpackstoff, wobei das Flächengewicht der Siegelschicht 1 bis 15 g/m² beträgt,
- Aktivierung des Siegelmediums **durch** einen Aufschmelzprozeß und Ausbildung einer haftklebenden Schicht.

3. Verwendung nach Anspruch 2, **dadurch gekennzeichnet*,* daß** das Flächengewicht der Siegelschicht 2,5 bis 3,5 g/m² beträgt.

## Claims

1. Sealing medium for composite packaging systems, in particular for packaging transdermal therapeutic systems (TTS) with volatile active ingredients, **characterized in that** said sealing medium is a heat sealing lacquer which is in the form of a liquid phase and contains an ethylene/methacrylic acid copolymer dispersion or is an ethylene/methacrylic acid copolymer dispersion and results in no measurable uptake of active ingredient.

2. Use of a sealing medium for the production of composite packaging systems, especially for transdermal therapeutic systems (TTS), **characterized by**
- providing a liquid heat sealing laquer which consists of an ethylene/methacrylic acid copolymer dispersion or contains an ethylene/methacrylic acid copolymer dispersion and which results in no measurable uptake of active ingredient,
- applying the liquid heat sealing laquer in a printing process onto a composite packaging material to form a sealing layer, the weight per unit area of the sealing layer being 1 to 15 g/m²,
- activating the sealing medium by means of a melting process and forming a pressure-sensitive adhesive layer.

3. Use according to claim 2, **characterized in that** the weight per unit area of the sealing layer is 2.5 to 3.5 g/m².

## Revendications

1. Agent de scellage pour systèmes d'emballage composites, en particulier pour l'emballage de systèmes thérapeutiques transdermiques (STT) avec des substances actives volatiles, **caractérisé en ce que** l'agent de scellage est une laque de scellage à chaud, qui se trouve en phase liquide et qui contient une dispersion de copolymère d'éthylène-acide méthacrylique ou qui est une dispersion de copolymère d'éthylène-acide méthacrylique, dans laquelle il ne se produit pas d'absorption mesurable de la substance active.

2. Utilisation d'un agent de scellage pour la fabrication de systèmes d'emballage composites, en particulier pour l'emballage de systèmes thérapeutiques transdermiques (STT), **caractérisée par**
- la préparation d'une laque de scellage à chaud liquide qui est constituée par une dispersion de copolymère d'éthylène-acide méthacrylique ou qui contient une dispersion de copolymère d'éthylène-acide méthacrylique, dans laquelle il ne se produit pas d'absorption mesurable de la substance active
- l'application de la laque de scellage à chaud liquide dans un procédé d'impression en une couche de scellage sur un emballage composite, le poids surfacique de la couche de scellage étant de 1 à 15 g/m²,
- l'activation de l'agent de scellage par un procédé de fusion et réalisation d'une couche auto-adhésive.

3. Utilisation selon la revendication 2, **caractérisée en ce que** le poids surfacique de la couche de scellage est de 2,5 à 3,5 g/m².
